# EUROPEAN PATENT APPLICATION

(11) **EP 3 809 414 A1**
(43) Date of publication of application: **21.04.2021**
(21) Application number: 19203270.4
(22) Date of filing: 15.10.2019
(51) Int. Cl.: G16B 40/30

(54) **METHOD AND APPARATUS FOR ASSESSING PATIENT'S RESPONSE TO THERAPY**

(71) Applicant: Koninklijke Philips N.V., 5656 AG Eindhoven (NL)
(72) Inventor: VAN DER ZAAG, Pieter Jan, 5656 AE Eindhoven (NL); VERHAEGH, Wilhelmus Franciscus Johannes, 5656 AE Eindhoven (NL)
(74) Representative: Philips Intellectual Property & Standards

(57) **Abstract**

The present invention relates to monitoring a patient's response to therapy. In order to facilitate monitoring a patient's response to therapy, a method is provided to identify groups of trends in each of genetic changes with similar genetic changes over time and to analyse these groups to determine the patient's response to therapy.

## Description

### FIELD OF THE INVENTION

The present invention relates to monitoring a patient's response to a cancer therapy, and in particular to a method, an apparatus, and a system for monitoring a patient's response to a cancer therapy, a computer program element, and a computer readable medium.

### BACKGROUND OF THE INVENTION

Oncologists usually use the images created by computed tomography (CT) and/or magnetic resonance imaging (MRI) scans to guide treatments and to compare a tumour to how it was before treatment began, aiding physicians to assess the patient's response to treatment. Although these imaging methods have the advantage of providing the location and shape (with respect to other organs, for example) of the tumour, it may be difficult to assess early on changes in tumour volume, as the resolution of the medical imaging is typically in the range of millimetre. Monitoring therapy response of a cancer patient may also be done by analysing patient's samples, such as a liquid biopsy including blood or other body liquid, and following the occurrence of specific mutations over time during treatment. Since tumours may harbour various mutations, a broad panel covering many different mutations may be investigated. Consequently, it may become very difficult to monitor the response of patients to their therapy as all these mutations should be tracked and analysed.

### SUMMARY OF THE INVENTION

There may be a need to facilitate monitoring a patient's response to therapy.

The object of the present invention is solved by the subject-matter of the independent claims, wherein further embodiments are incorporated in the dependent claims. It should be noted that the following described aspects of the invention apply also for the method, the apparatus, the system, the computer program element, and the computer readable medium.

A first aspect of the invention relates to a method of monitoring a patient's response to a cancer therapy, comprising the following steps:
a) receiving data indicative of occurrences of genetic changes over time in a patient, each of the genetic changes being associated with a common disease or a common disease group, wherein the data is obtained based on an analysis of a biomarker in a patient sample;
b) grouping trends in each of the genetic changes into a plurality of trend groups according to a similarity between the trends such that each trend group comprises trends representing similar temporal behaviours of the genetic changes' allele frequency, AF, wherein a similarity measure that quantifies the similarity between the trends in the same trend group lies within a pre-determined range; and
c) analysing the plurality of trend groups for monitoring the patient's response to the cancer therapy.

Since cancer may be a very heterogeneous disease, cancer patients may harbour many different mutations in their DNA. Hence, monitoring response to therapy across a broad patient group, e.g., using a liquid biopsy, may require a panel that covers many different mutations. As a consequence, it may become very difficult to monitor the response of patients to their therapy as all of these mutations should be tracked.

To solve this problem, a simple model is proposed in the present disclosure to reduce this complexity, even when complex panels with many different mutations (e.g., above forty different mutations) are used. To monitor therapy response of cancer patients to a therapy, such as a drug therapy, a radiation therapy, an immunotherapy, and/or a surgical procedure, one or more biomarkers may be extracted from a patient's sample, such as blood or other body liquid. Examples of the biomarkers include circulating tumour DNA (ct-DNA), which originates from dying (e.g. necrotic or apoptotic) tumour cells, and circulating tumour cells (CTCs), which are shed into the bloodstream from primary tumours and metastases. Currently, the ct-DNA and CTCs are clinically the most relevant biomarkers used to monitor therapy response. Based on an analysis of the one or more biomarkers in the blood sample, the occurrence of genetic changes over time in individual patients may be determined and stored in a dataset. Each of the genetic changes is associated with a common disease or a common disease group. In other words, various genetic changes relevant to the disease or the common disease group, e.g., breast cancer, may be tracked.

The genetic changes may also be referred to as mutations, i.e., a change in a genetic sequence. Mutations include changes as small as the substitution of a single DNA building block, or nucleotide base, with another nucleotide base. Meanwhile, larger mutations can affect many genes on a chromosome. Along with substitutions, mutations can also be caused by insertions, deletions, or multiplications of DNA sequences. Although some mutations are hereditary because they are passed down to an offspring from a parent carrying a mutation through the germ line, meaning through an egg or sperm cell carrying the mutation, the genetic changes in the present disclosure relate to nonhereditary mutations that occur in cells outside of the germ line, which are called somatic mutations. For example, some potent somatic mutations can cause cancer that will affect a single organism's survival.

Once the occurrence of genetic changes over time in individual patients is determined, a classification method, such as a clustering analysis or a self-organizing map, may be used to identify trend groups with trends having similar genetic changes over time, i.e. group-specific temporal behaviours of genetic changes. Each trend group comprises trends representing similar temporal behaviours of an allele frequency of the genetic changes. The allele frequency represents the incidence of a gene variant in a population. Alleles are variant forms of a gene that are located at the same position, or genetic locus, on a chromosome. An allele frequency is calculated by dividing the number of times the allele of interest is observed in a population by the total number of copies of all the alleles at that particular genetic locus in the population. Allele frequencies may be represented as a decimal, a percentage, or a fraction. Changes in allele frequencies over time can indicate that genetic drift is occurring or that new mutations have been introduced. It is also noted that with respect to changes in the popution genetic germ line variants, the term "allele frequency" may also be refered to as variant allele frequency (VAF), for instance for the variants giving rise to colour of hair, eyes. etc. With respect to tumour-induced mutations, the term "allele frequency" may also be referred to as mutant allele frequency (MAF). As the genetic changes in the present disclosure relate to tumour-induced mutations, the allele frequency in the present disclosure may also be referred to as MAF.

These identified trend groups are then further analysed for monitoring the patient's response to the cancer therapy. In other words, these identified trend groups are associated with characteristics related to patient's response to a cancer therapy. That is, there is no need to analyse a large dataset of genetic changes being tracked. Rather, only trend groups are analysed for determining the patient's response to a cancer therapy. As the number of trend groups is much less than the number of genetic changes being monitored, the complexity is reduced even when having to use complex panels with many different mutations, e.g., larger than forty mutations.

It is also noted that this analysis yields a technical result or a technical intermediate result that is of use to a physician to subsequently reach the diagnosis in *stricto sensu.* I.e. the analysis step c) does not actually produce a decision without the need of the physician's expertise.

According to an embodiment of the present invention, in step b) trends are grouped using at least one of the following methods:
(i) performing a clustering analysis to identify different trend groups of trends;
(ii) using a self-organizing map to identify different trend groups of trends; and
(iii) comparing trends with a pre-defined trend grouping rule to associate each trend with a respective trend group.

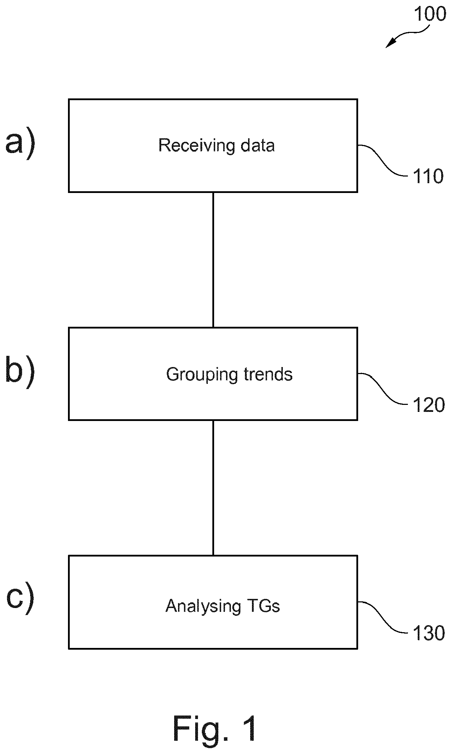

This will be explained hereafter and particularly with respect to the exemplary embodiment illustrated in Fig. 1.

According to an embodiment of the present invention, step c) further comprises:
- dividing the trend groups into a plurality of trend classes based on a trend classification rule, each trend class being associated with a respective patient's response to a treatment, such that each trend class comprises trend groups having trends representing the same patient's response to a treatment, wherein the trend classification rule defines the trend classes in terms of the temporal behaviours of the genetic changes;
- applying a trend score assigning rule to assign a trend score to each of the trend groups based on its associated trend class, wherein the trend score assigning rule defines the trend scores in terms of the trend classes; and
- determining a worst-case trend score of the genetic changes for being used to determine a response state of the cancer patient.

In other words, it is proposed to classify trends in each of the genetic changes over time and to summarize these into a single measure of therapy response. More specifically, a trend classification rule is used to identify trend classes of trend groups having trends representing similar trend-impact, e.g., positive or negative, on patient's response to a treatment. Accordingly, the number of trend classes representing the trend-impact is much less than the number of trend groups and, of course, also the number of genetic changes being monitored for the disease, e.g., breast cancer. Then, these trend classes are assigned a trend-score according to a trend score assigning rule. For an individual patient, the worst-case trend score is computed. The worst-case trend score corresponds to the trend score that represents the most negative trend-impact on the patient's response to the treatment. Finally, patients are assessed or stratified according to the computed worst-case trend score.

In this way, a more reliable and easier accessible discrimination may be provided between different patient's responses to the treatment, such as effectively responding, partial responding, non-responding (also referred to as stable disease), or progressive disease for each individual patient. The computation of the worst-case trend score for individual patients from monitored biomarkers may be carried out without human supervision or interference.

According to an embodiment of the present invention, the trend classification rule classifies the trends into the following categories:
(i) an AF dropping to 0 and not rising later classified as trend class complete response, CR;
(ii) an AF dropping to less than a predefined percentage of its initial value and staying below this value in a time course classified as trend class partial response, PR;
(iii) an AF rising from its initial value to a predefined threshold and staying above the predefined threshold towards the end of a time course classified as trend class progressive disease, PD; and
(iv) the remaining trends in AF classified as trend class stable disease, SD.

For example, the predefined percentage of its initial value may range between 10% and 90% such as 10%, 20%, 30%, 40%, 50%, 60%, 70%, 80%, and 90%.

For example, the predefined threshold of the AF may range between 10% and 90% such as 10%, 20%, 30%, 40%, 50%, 60%, 70%, 80%, and 90%.

For example, the predefined percentage and/or the predefined threshold of the AF may be defined by a user.

This will be explained hereafter and particularly with respect to the exemplary embodiments illustrated in Fig. 1.

According to an embodiment of the present invention, the trend score assigning rule defines the following correspondence between the trend scores and the trend classes:
(i) a first score assigned to the trend class CR;
(ii) a second score assigned to the trend class PR;
(iii) a third score assigned to the trend class SD; and
(iv) a fourth score assigned to the trend class PD.

The first, second, third, and fourth scores have values in ascending order. Alternatively, the first, second, third, and fourth scores have values in descending order.

In an example, the first, second, third, and fourth scores maybe 0, 1, 2, and 3, respectively, thus having values in ascending order.

In another example, the first, second, third, and fourth scores may be 3, 2, 1, and 0, respectively, thus having values in descending order.

According to an embodiment of the present invention, the worst-case trend score is given by an extreme value of the trend scores of said genetic changes, and the patient's response state is categorised into the following classes:
(i) if the extreme value of the trend scores is equal to the first score, patient's CR is determined;
(ii) if the extreme value of the trend scores is equal to the second score, patient's PR is determined;
(iii) if the extreme value of the trend scores is equal to the third score, patient's SD is determined; and
(iv) if the extreme value of the trend scores is equal to the fourth score patient's PD is determined.

The extreme value corresponds to a maximum value of the trend scores, if the first, second, third, and fourth scores have values in ascending order. Alternatively, the extreme value corresponds to a minimum value of the trend scores, if the first, second, third, and fourth scores have values in descending order.

For example, if the first, second, third, and fourth scores are 0, 1, 2, and 3, respectively, and the maximum value of the trend scores is 2, it is determined that the patient response to the treatment is stable disease.

On the other hand, if the first, second, third, and fourth scores are 3, 2, 1, and 0, respectively, and the minimum value of the trend scores is 2, then it is determined that the patient response to the treatment is partial response.

According to an embodiment of the present invention, step c) further comprises counting a number of the trend groups to estimate a number of different tumour sub-clones.

In other words, it is proposed to use a classification method, such as a clustering analysis or a self-organizing map, to identify different groups of trends to determine the number of different time courses. This may give an estimation of the number of different tumour sub-clones to assess intra-tumour heterogeneity, as sub-clones respond differently to certain treatments. This may be beneficial for deciding on a therapy regimen, and changes in heterogeneity may be a trigger to adapt therapy for optimal therapy choice.

According to an embodiment of the present invention, step c) further comprises estimating, for each of the sub-clones, a relative fraction of a tumour that it covers, at each point in time.

For example, the fraction of the tumour a particular sub-clone may cover may be around the following two bounds:
The fraction of a tumour that is the tumour devided by the number of sub-clones found.

The fraction of the sum of the MAF (%) of this sub-clone with respect to the sum of all MAF (%) for all mutants.

According to an embodiment of the present invention, the biomarker comprises at least one of circulating tumour DNA, ct-DNA, and genetic analyses of circulating tumour cells, CTCs.

Currently, the ct-DNA and CTCs are clinically the most relevant biomarkers used to monitor therapy response. Of these, ct-DNA may be the one which is mostly pursued, as CTCs may occur at low count in most cancer patients unless they have metastatic disease.

According to an embodiment of the present invention, the method further comprises outputting at least one of the following into a clinical decision support system:
- the number of different tumour sub-clones;
- for each of the tumour sub-clones, a relative fraction of a tumour that it covers, at each point in time; and/or
- the worst-case trend score.

The number of different tumour sub-clones and the relative fraction of a tumour that it covers at each point in time may be used provide additional information to clinician to assess tumour heterogeneity.

The worst-case trend score may be a trigger to perform medical imaging, such as MRI or CT, to provide further information concerning the patient's response to therapy. In some examples, the output may be combined with medical imaging to improve the efficiency and accuracy of monitoring patient's response to therapy.

A second aspect of the present invention relates to a decision-support apparatus for monitoring a patient's response to a cancer therapy. The decision-support apparatus comprises an input unit and a processing unit. The input unit is configured for receiving data indicative of an occurrence of genetic changes over time in a patient, each of the genetic changes being associated with a common disease or a common disease group. The data has been obtained based on an analysis of a biomarker in a patient sample. The processing unit configured for performing a method as describe above and below.

The term "unit" as used herein may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality.

In some examples, the processing unit is a single unit to perform steps b) and c).

In some examples, the processing unit comprises two subunits, one for performing step b) and the other for performing step c). The subunits may integrated into a single processing unit. Alternatively, the subunits may be at different locations to perform distributed computing.

A third aspect of the present invention relates to a system for monitoring a patient's response to a cancer therapy. The system comprises a decision-support apparatus as described above and below and a sample analysing device configured for analysing a patient sample to obtain data indicative of an occurrence of genetic changes over time in the patient to be output to the apparatus.

In some examples, the decision-support apparatus may be integrated with the sample analysing device. For example, the computing unit of the sample analysing device may be further configured to perform the method steps as described above and below.

In some examples, the decision-supported apparatus may be provided separately from the sample analysing device. For example, the decision-support apparatus may be coupled to the sample analysing device via a physical cable or wirelessly to receive data.

Examples of the sample analysing device may include, but not limited to, sequencers such as the HiSeq of Illumina or the IonTorrent of ThermoFisher, mass-spectrometry systems such as the Agena Biosciences MassARRAY system, a digital droplet polymerase chain reaction (PCR) system such as the Bio-Rad QX200 droplet digital PCR, and/or a multiplex PCR system such as the Biocartis Idylla platform.

According to an embodiment of the present invention, the system further comprises a medical imaging apparatus for monitoring the patient's response to a cancer therapy by imaging.

Examples of the medical imaging apparatus include, but not limited to, MRI, CT, and positron-emission tomography (PET) imaging apparatus. The combination of therapy response monitoring by imaging and through patient's samples, e.g., liquid biopsy, may improve the efficiency and accuracy for monitoring patient's response to therapy.

According to another aspect of the present invention, it is provided a computer program element for controlling an apparatus as described above and below, which, when being executed by a processing unit, is adapted to perform the method steps as described above and below.

According to a further aspect of the present invention, it is provided a computer readable medium having stored the program element.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 shows a flow diagram of a method for monitoring a patient's response to a cancer therapy according to an exemplary embodiment of the present disclosure.
Fig. 2 shows some examples of biomarkers, which may be extracted from a blood draw.
Fig. 3 shows a flow diagram of a method for monitoring a patient's response to a cancer therapy according to another exemplary embodiment of the present disclosure.
Figs. 4A, 4B, and 4C show exemplary examples from literature that are representative for various types of dynamics of ct-DNA.
Fig. 5 shows a flow diagram of a method for monitoring a patient's response to a cancer therapy according to a further exemplary embodiment of the present disclosure.
Fig. 6 shows an example of five trends in genetic changes A-E, which are tracked over five time points.
Fig. 7 shows a decision-support apparatus for monitoring a patient's response to a cancer therapy according to an exemplary embodiment of the present disclosure.
Fig. 8 shows a system for monitoring a patient's response to a cancer therapy according to an exemplary embodiment of the present disclosure.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

Monitoring therapy response of a cancer patient may be done by using medical imaging equipment, such as MRI and CT. Although these methods have the advantage of providing the location of the tumour, it is not always easy to assess early on changes in tumour volume, as the resolution of medical imaging is typically in the range of millimetre. Another method for monitoring therapy response of a cancer patent maybe done by analysing biomarkers, such as circulating tumour DNA, in patient's samples, such as blood or other body fluid, and following the occurrence of specific mutations over time during treatment. However, since tumours may harbour various mutations, a broad panel covering many different mutations is typically investigated, which may show different trends over time. Consequently, it may be very difficult to monitor the response of patients to their therapy.

In order to facilitate monitoring the patient's response to a cancer therapy, the following disclosure describes the present invention according to several embodiments directed at methods, apparatus, systems, computer programme elements and computer readable medium related to the personalized assessment of therapy response using a dataset representing the dynamics of the occurrence of genetic changes in individual patients and a statistical classification model, such as a clustering analysis or a self-organizing map. More specifically, the techniques described herein process the data using a statistical classification model to identify group of trends with similar genetic changes over time, and associate these groups with characteristics related to patient's response to a cancer therapy. As the number of trend groups is much less than the number of genetic changes to be tracked, the determination of the patient's response maybe greatly facilitated.

Fig. 1 illustrates a flow diagram of a method 100 for monitoring a patient's response to a cancer therapy. Examples of cancer therapy may include, but not limited to, a drug therapy, a radiation therapy, an immunotherapy, and/or a surgical procedure. This method may also be applied for non-invasive prenatal testing or infections disease monitoring.

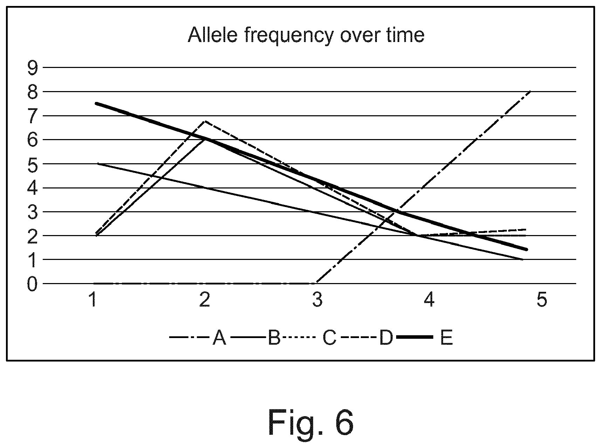

In step 110, i.e., step a), data indicative of an occurrence of genetic changes over time in a patient is received, e.g., by a decision-support apparatus as illustrated in Fig. 6. Each of the genetic changes is associated with a common disease or a common disease group. In other words, various genetic changes relevant to a disease or a common disease group, e.g., breast cancer, may be tracked. The data may have been obtained based on an analysis of a biomarker in a patient sample, e.g., a liquid biopsy, and stored in a dataset. For example, biomarkers may be extracted from a blood draw.

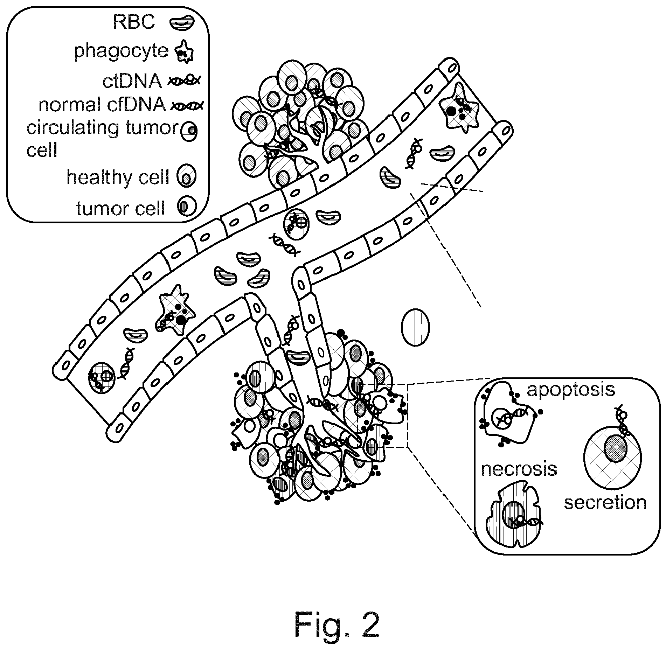

Fig. 2 is adapted from a figure on page "Circulating tumor DNA" in Wikipedia showing some examples of biomarkers, which may be extracted from the blood, including red blood cell (RBC), phagocyte, circulating tumour DNA (ct-DNA), normal circulating free DNA (cf-DNA), circulating tumour cell (CTC), healthy cell, and tumour cell. Tumours may release multiple biomarkers in the bloodstream, such as ct-DNA, CTCs, exosomes, and platelets. Ct-DNA originates from dying tumour cells. CTCs are shed into the bloodstream from primary tumours and metastases. Exosomes are cell-derived vesicles containing tumour messenger RNA (mRNA), microRNA (miRNA), protein, and dsDNA. Platelets are reported to pick up tumour RNA in circulation. Currently, ct-DNA and CTCs may be clinically the most relevant biomarkers used to monitor therapy response. Of these, ct-DNA may be commonly used, as CTCs may occur at low count (e.g., less than 10) in most cancer patients unless they have metastatic disease.

Since different cancer patients may carry different mutations in their tumours, one may need to monitor a large panel of possible genetic changes, i.e., mutations and/or epigenetic changes, in order to assure that at least some of them can be found in the biomarker, such as ct-DNA. For example, breast cancer is a very heterogeneous disease with a wide range of genetic changes that may occur, yet all with a limited frequency among the patient population, e.g., at 15% or less. For example, an analysis of the various genetic changes relevant to breast cancer and their typical occurrence in patients is illustrated in Table 1:

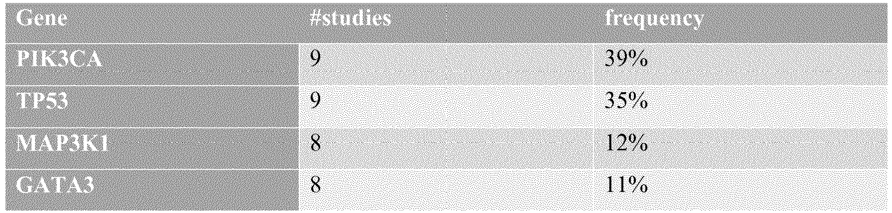

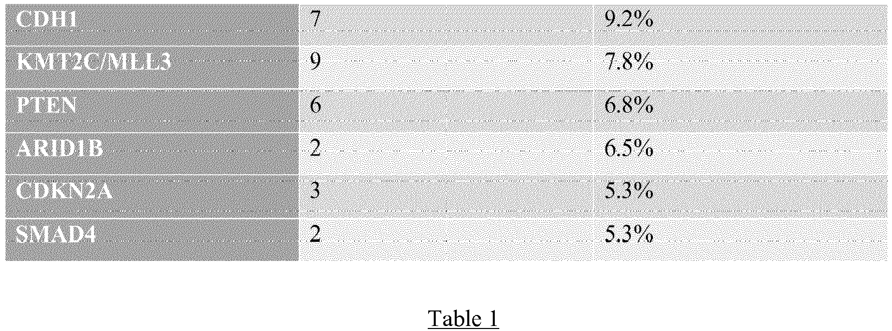

In addition, several hotspots may need to be monitored within these genes, resulting that at least forty-four positions in the biomarker need to be monitored. In certain cases, this may be insufficient, and some additional twelve sites may need to be monitored for epigenetic changes. Furthermore, also the occurrence of copy number variations (CNVs), such as the well-known HER2 CNV, may need to be examined.

In step 120, i.e., step b), trends in each of the genetic changes, i.e., changes in allele frequencies (AF) over time for each of the genetic changes, are grouped into a plurality of trend groups, also referred to as TGs, according to a similarity between these trends. Each trend group comprises trends representing similar temporal behaviours of the genetic changes. In other words, this step identifies groups of trends with a similar temporal behaviour pattern, i.e., similar changes in allele frequencies over time. The similarity between the trends is quantified by a similarity measure. In the same trend group, the similarity measure lies within a pre-determined range.

Various methods may be used to identify groups of trends with group-specific temporal behaviour patterns. For example, a clustering analysis maybe performed to identify different groups of trends. Clustering analysis, or clustering, is an unsupervised learning technique, which aims at grouping a set of trends into clusters, i.e., groups, so that trends in the same clusters should be similar as possible, whereas trends in one cluster should be as dissimilar as possible from trends in other clusters. Clustering analysis aims to group a collection of patterns into clusters based on similarity. A typical clustering technique uses a similarity function for comparing various data items. In particular, clustering is done based on a similarity measure to group similar data objects together. This similarly measure may be based on distance functions such as Euclidean distance, Manhattan distance, Minkowski distance, Cosine similarity, etc. to group trends in clusters. The clusters may be formed in such a way that any two trends within a cluster have a minimum distance value and any two trends across different clusters have a maximum distance value. In other words, the similarity measure between the trends in the same trend group lies within a pre-determined range. In another example, a self-organizing map be used to identify different trend groups of trends. A self-organizing map or self-organizing feature map is a type of artificial neural network (ANN) that is trained using unsupervised learning to produce a low-dimensional, discretized representation of the input space of the training samples, called a map according to a similarity measure, such as Euclidean distance. In a further example, trends may be compared with a pre-defined trend grouping rule to associate each trend with a respective trend group. For example, the pre-defined trend grouping rule may specify that trends with an AF that drops to 0 and does not rise later are grouped in the same group.

In step 130, i.e., step c), the plurality of trend groups is analysed for monitoring the patient's response to the cancer therapy. In other words, this step associates these trend groups with characteristics related to the patient's response to the cancer therapy. Grouping trends into trend groups may compress a broad panel of genetic changes to be tracked in to simpler, yet representative, groups of trends with group-specific temporal behaviour patterns, thus facilitating the analysis of the trends for monitoring the patient's response to a cancer therapy. It is also noted that .the analysis step c) does not actually produce a decision without the need of the physician's expertise. In other words, this analysis yields a technical result or a technical intermediate result that is of use to a physician to subsequently reach the diagnosis in *stricto sensu.*

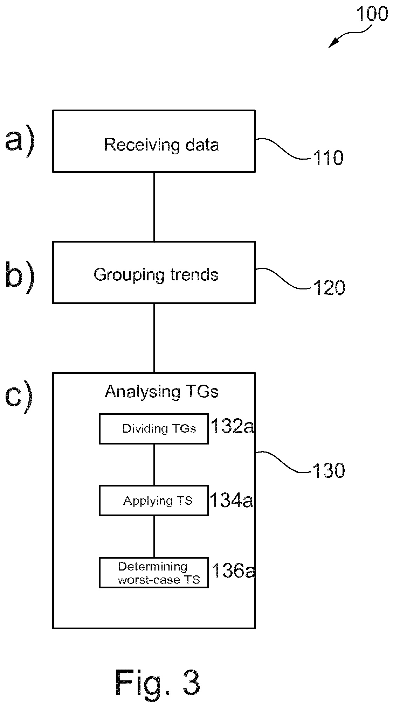

In an example, the plurality of trend groups may be analysed for determining whether a patient is responding or not to the therapy. As previously mentioned, a great multitude of genetic changes need to be measured in all patients as one does not known in which patient such mutations occur and in some patients new mutations may occur as the disease progresses, such as resistance genes. As a consequence, it may become very difficult to monitor the response of the patients to their therapy as all of these mutations should be tracked. As will be explained hereafter and particularly with the exemplary embodiment in Fig. 3, it is proposed to further analyse the plurality of trend groups and to summarize the trends of a large set of genetic changes into a single response score. In this way, a simple model is proposed to reduce the complexity that even when having to use complex panels with many different mutations, e.g., larger than forty mutations, and a simple metric is deduced to determine whether a patient is responding or not to the therapy.

Fig. 3 illustrates a flow diagram of a method 100 for monitoring a patient's response to a cancer therapy according to this example. In the illustrated flow diagram, step 130, i.e., step c), further comprises the following steps:

In step 132a, the trend groups are divided into a plurality of trend classes based on a trend classification rule. Each trend class is associated with a respective patient's response to a treatment, such that each trend class comprises trend groups having trends representing the same patient's response to a treatment. The trend classification rule defines the trend classes in terms of the temporal behaviours of the genetic changes. In other words, this step uses a trend classification rule to further group the trend groups into trend classes, and to associate these trend classes with characteristics related to different patient's response to a treatment. For example, the trend classification rule may classify the trends into the following categories: (i) an AF dropping to 0 and not rising later classified as trend class complete response (CR), (ii) an AF dropping to less than a predefined percentage of its initial value and staying below this value in a time course classified as trend class partial response (PR), (iii) an AF rising from its initial value to a predefined threshold and staying above the predefined threshold towards the end of a time course classified as trend class progressive disease (PD), and (iv) the remaining trends in AF classified as trend class stable disease (SD).

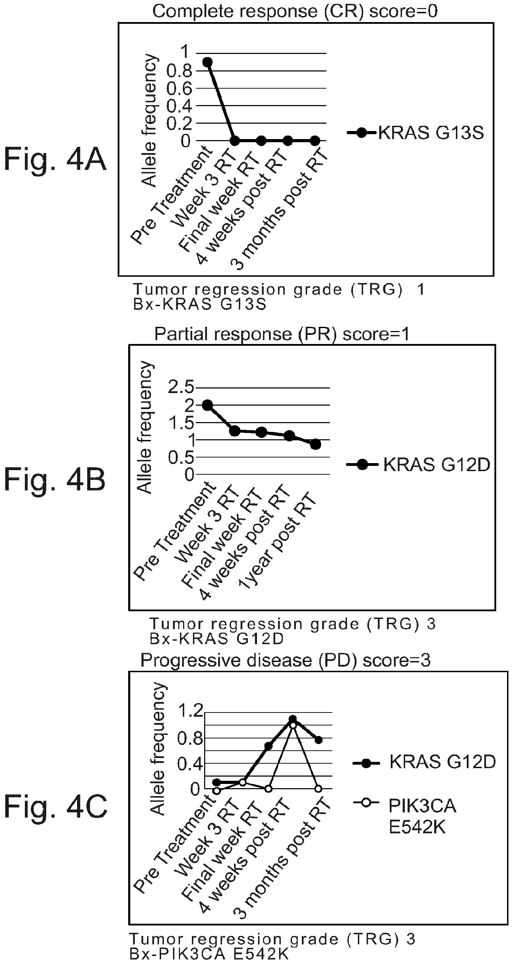

Figs. 4A, 4B, and 4C show exemplary examples from literature that are representative for various types of dynamics of ct-DNA in form of the fraction of the mutants with respect to the normal cells, e.g., normal wild-type cells, in a patient's blood. The data are taken from the poster presentation by S. Toomey et al. entitled "Non-invasive genotyping of locally advanced rectal cancer patients using circulating tumour DNA" which was presented at the International Symposium on Minimal Residual Cancer (ISMRC) held on May 3-5, 2018 in Dublin, Ireland. Depending on the sensitivity of the technique employed, this may be measured between 0.001 and 2 or 3 %, with exceptional cases reaching 10 %. The above-mentioned trend classification rule may be used to identify to which of a set of trend classes these exemplary examples belong. For example, in Fig. 4A, the curve shows that the AF drops at the third week of treatment and does not rise later. The term "Bx" stands for the mutation identified in the pre-treatment biopsy. Note that this may be the same mutation as typically tracked with the liquid biopsies. Accordingly, the trend in Fig. 4A is classified as CR. In Fig. 4B, the curve shows that the AF drops to less than a predefined percentage, and stays below this value in a time course. For example, the predefined percentage of its initial value may range between 10% and 90% such as 10%, 20%, 30%, 40%, 50%, 60%, 70%, 80%, and 90%. A user may define the predefined percentage. Accordingly, the trend in Fig. 4B is classified as PR. In Fig. 4C, the upper curve shows that the AF rises from its initial value to a predefined threshold, and stays above the predefined threshold towards the end of the time course. Accordingly, the trend represented by the upper curve is classified as PD. On the other hand, the trend represented by the lower curve shows that the AF is classified as SD, as it is none of the above patterns. For example, the predefined threshold of the AF may range between 10% and 90% such as 10%, 20%, 30%, 40%, 50%, 60%, 70%, 80%, and 90%. The predefined threshold of the AF may be defined by a user.

In step 134a, a trend score assigning rule is applied to assign a trend score, also referred to as TS, to each of the trend groups based on its associated trend class. The trend score assigning rule defines the trend scores in terms of the trend classes. This step links the trend classes to trend scores. In other words, for each of the trends in the allele frequency, i.e., the occurrence of the mutated allele with respect to the normal or wild type allele, a corresponding score is introduced.

Turning to Figs. 4A, 4B, and 4C, the trend score assigning rule may define the following correspondence between the trend scores and the trend classes: (i) a first score assigned to the trend class CR, (ii) a second score assigned to the trend class PR, (iii) a third score assigned to the trend class SD, and (iv) a fourth score assigned to the trend class PD. The first, second, third, and fourth scores may have values in ascending order, such as 0, 1, 2, and 3, as illustrated in Figs. 4A to 4C. In other words, this may be indicated below namely: 0 for response, 1 for partial response, 2 for stable disease, and 4 for progressive disease. Alternatively, the first, second, third, and fourth scores may have values in descending order, such as 3, 2, 1, and 0. In other words, this may be indicated below namely: 3 for response, 2 for partial response, 1 for stable disease, and 0 for progressive disease.

In step 136a, a worst-case trend score of the genetic changes is determined for being used to determine a response state of the cancer patient. For example, the worst-case trend score may be given by an extreme value of the trend scores of said genetic changes. The patient's response state may be categorized into the following classes: (i) if the extreme value of the trend scores is equal to the first score, patient's CR is determined; (ii) if the extreme value of the trend scores is equal to the second score, patient's PR is determined; (iii) if the extreme value of the trend scores is equal to the third score, patient's SD is determined; and (iv) if the extreme value of the trend scores is equal to the fourth score patient's PD is determined.

In an example, the extreme value may correspond to a maximum value of the trend scores, if the first, second, third, and fourth scores have values in ascending order, such as 0, 1, 2, and 3. In other words, the final score may be classified such that we get a value e.g., between 0-3 putting the patients in any of the four overall classes identified: (i) 0 = patient's complete response, (ii) 1 = patient's partial response, (iii) 2 = patient's stable disease, and (iv) 3 = patient's progressive disease.

In another example, the extreme value may correspond to a minimum value of the trend scores, if the first, second, third, and fourth scores have values in descending order, such as 3, 2, 1, and 0. In other words, the final score may be classified such that we get a value e.g., between 0-3 putting the patients in any of the four overall classes identified: (i) 3 = patient's complete response, (ii) 2 = patient's partial response, (iii) 1 = patient's stable disease, and (iv) 0 = patient's progressive disease.

In the exemplary example in Figs. 4A, 4B, and 4C, the final score is 3. Thus, a patient's PD is determined.

Accordingly, this approach compresses the data space of a large dataset of monitored genetic changes into simpler, yet representative, response-scores. In this way, the number of trend-scores representing the trend-impact is much less than the number of genetic changes being monitored for the disease, e.g., breast cancer. Hence, the method may provide a more reliable and easier accessible discrimination between effectively responding, partially responding, non-responding (i.e., stable disease), or progressive disease for each individual patient. In addition, the computation of the worst-case score, e.g., the extreme trend score, for individual patients from the monitored biomarkers may be carried out without human supervision or interference.

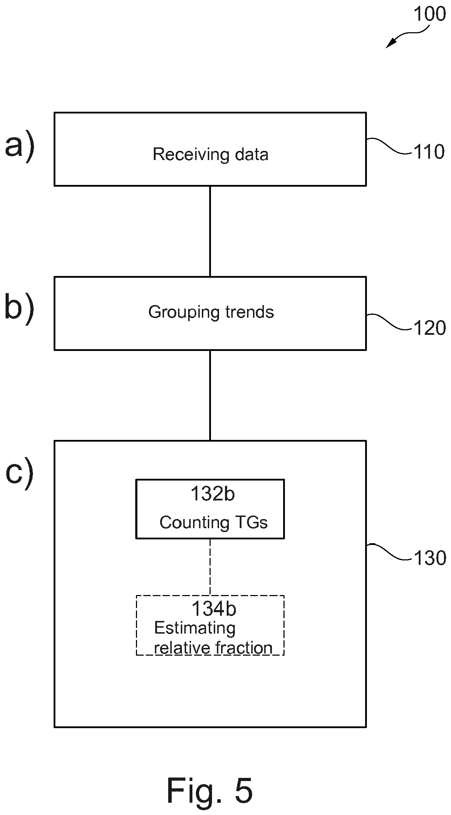

In another example, the plurality of trend groups may be analysed for accessing intra-tumour heterogeneity. Intra-tumour heterogeneity implies that a cancer patient's tumour does not consist of one type of cancer cells, but that multiple sub-clones exist and each have their own characteristics and that respond differently to certain treatments. Assessing tumour heterogeneity is therefore important to decide on a therapy regimen, and changes in heterogeneity may be a trigger to adapt therapy. A heterogeneity analysis of the original tumour may not be sufficient for this, and analysis of the biomarker, such as ct-DNA, at certain time point may also be insufficient to give sufficient insight. As will be explained hereafter and particularly with the exemplary embodiment in Fig. 5, it is proposed to further analyse the trend groups to make an assessment of the tumour heterogeneity, which is relevant for optimal therapy choice.

Fig. 5 illustrates a flow diagram of a method 100 for monitoring a patient's response to a cancer therapy according to this example. In the illustrated flow diagram, step 130, i.e., step c), further comprises the following steps:
In step 132b, the number of the trend groups is counted to estimate a number of different tumour sub-clones. For example, Fig. 6 shows an example of five trends in genetic changes A-E, which are tracked over five time points. Basically, there are three different trend groups: trend B and trend E only have a scale factor difference and belong to an trend group; trend C and trend D belong to another trend group; and trend A alone belongs to a further trend group. Hence, it may be concluded that there are three sub-clones, of which two are present at times 1 to 3, and three at times 4 and 5.

Optionally, step 130 may further comprise step 134b, in which it may be estimated for each of the sub-clones the relative fraction of tumours that it covers, at each point in time. This maybe estimated using the following two bounds: the fraction of a tumour that is the tumour divided by the number of sub-clones found, and the fraction of the sum of the MAF (%) of this sub-clone with respect to the sum of all MAF (%) for all mutants.

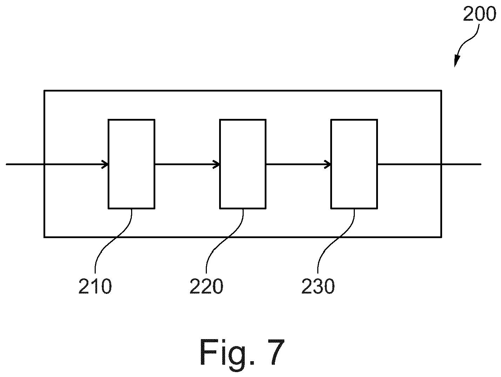

Fig. 7 shows a decision-support apparatus 200 for monitoring a patient's response to a cancer therapy according to some embodiments of the present disclosure. The system 200 comprises an input unit 210, and a processing unit 220.

The input unit 210 is configured for receiving data indicative of an occurrence of genetic changes over time in a patient. Each of the genetic changes being associated with a common disease or a common disease group. The data is obtained based on an analysis of a biomarker in a patient sample.

The processing unit 220 is configured for performing any one of the above-mentioned method steps. For example, the processing unit 220 may be configured to perform any one of methods illustrated in Figs. 1, 3, and 5.

Optionally, as illustrated in Fig. 7, the decision-support apparatus may further comprise an output unit 230 for outputting the results. In an example, the output unit 230 may comprise a display.

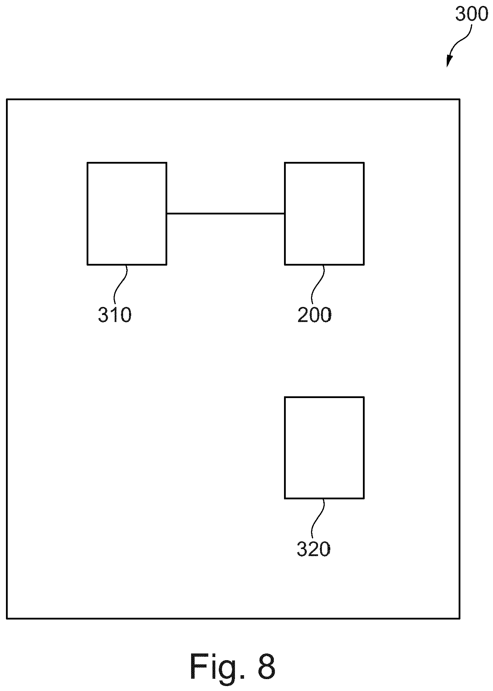

Fig. 8 shows a system 300 for monitoring a patient's response to a cancer therapy according to some embodiments of the present disclosure. The system 300 comprises a decision-support apparatus 200 as described above and a sample analysing device 310.

The sample analysing device 310 is configured for analysing a patient sample to obtain data indicative of an occurrence of genetic changes over time in the patient to be output to the apparatus. In an example, the sample analysing device 310 may be sequencers, such as the HiSeq of Illumina or the IonTorrent of ThermoFisher. In another example, the sample analysing device 310 may be mass-spectrometry systems, such as the Agena Biosciences MassARRAY system. In a further example, the sample analysing device 310 may be a digital droplet polymerase chain reaction (PCR) system, such as the Bio-Rad QX200 droplet digital PCR. In a further example, the sample analysing device 310 may be a multiplex PCR system, such as the Biocartis Idylla platform.

Optionally, the system may further comprise a medical imaging apparatus 320 for monitoring the patient's response to a cancer therapy by imaging. In other words, monitoring therapy response using patient's samples may be combined with monitoring therapy response in oncology patient by imaging. Examples of the medical imaging apparatus may include, but not limited to, a magnetic resonance imaging (MRI) apparatus, an X-ray imaging apparatus, a computed tomography (CT) imaging apparatus, an ultrasound (US) imaging apparatus, and a positron-emission tomography (PET) imaging apparatus. The medical imaging methods have the advantage of providing the location of the tumour, but it is not always easy to assess change in tumour volume, as the resolution of medical imaging is typically in the range of millimetre. On the other hand, biomarkers, such as ct-DNA, provides molecular information concerning which mutations in a patient's DNA maybe the cause of the cancer, and hence which drug to give. Moreover, as biomarkers measure on a molecular level, the response can be detected much sooner than by imaging. Furthermore, biomarkers may provide further information, such as the genetic profile of the tumour, tumour heterogeneity and resistance to therapy. The combination of both methods for monitoring a patient's response to a cancer therapy may thus provide a more efficient and accurate way to monitor the progress, response and/or tumour heterogeneity over a whole time course.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A method (100) of monitoring a patient's response to a cancer therapy, comprising the following steps:
a) receiving (110) data indicative of an occurrence of genetic changes over time in a patient, each of the genetic changes being associated with a common disease or a common disease group, wherein the data is obtained based on an analysis of a biomarker in a patient sample;
b) grouping (120) trends in each of the genetic changes into a plurality of trend groups according to a similarity between the trends such that each trend group comprises trends representing similar temporal behaviours of the genetic changes' allele frequency, AF, wherein a similarity measure that quantifies the similarity between the trends in the same trend group lies within a pre-determined range; and
c) analysing (130) the plurality of trend groups for monitoring the patient's response to the cancer therapy.

2. Method according to claim 1,
wherein in step b) trends are grouped using at least one of the following methods:
- performing a clustering analysis to identify different trend groups of trends;
- using a self-organizing map to identify different trend groups of trends; and
- comparing trends with a pre-defined trend grouping rule to associate each trend with a respective trend group.

3. Method according to claim 1 or 2,
wherein step c) further comprises:
- dividing (132a) the trend groups into a plurality of trend classes based on a trend classification rule, each trend class being associated with a respective patient's response to a treatment, such that each trend class comprises trend groups having trends representing the same patient's response to a treatment, wherein the trend classification rule defines the trend classes in terms of the temporal behaviours of the genetic changes;
- applying (134a) a trend score assigning rule to assign a trend score to each of the trend groups based on its associated trend class, wherein the trend score assigning rule defines the trend scores in terms of the trend classes; and
- determining (136a) a worst-case trend score of the genetic changes for being used to determine a response state of the cancer patient.

4. Method according to claim 3,
wherein the trend classification rule classifies the trends into the following categories:
(i) an AF dropping to 0 and not rising later classified as trend class complete response, CR;
(ii) an AF dropping to less than a predefined percentage of its initial value and staying below this value in a time course classified as trend class partial response, PR;
(iii) an AF rising from its initial value to a predefined threshold and staying above the predefined threshold towards the end of a time course classified as trend class progressive disease, PD; and
(iv) the remaining trends in AF classified as trend class stable disease, SD.

5. Method according to claim 4,
wherein the trend score assigning rule defines the following correspondence between the trend scores and the trend classes:
(i) a first score assigned to the trend class CR;
(ii) a second score assigned to the trend class PR;
(iii) a third score assigned to the trend class SD; and
(iv) a fourth score assigned to the trend class PD;
wherein the first, second, third, and fourth scores have values in ascending order or in descending order.

6. Method according to claim 5,
wherein the worst-case trend score is given by an extreme value of the trend scores of said genetic changes, and the patient's response state is categorised into the following classes:
(i) if the extreme value of the trend scores is equal to the first score, patient's CR is determined;
(ii) if the extreme value of the trend scores is equal to the second score, patient's PR is determined;
(iii) if the extreme value of the trend scores is equal to the third score, patient's SD is determined; and
(iv) if the extreme value of the trend scores is equal to the fourth score patient's PD is determined;
wherein the extreme value corresponds to:
- a maximum value of the trend scores, if the first, second, third, and fourth scores have values in ascending order; or
- a minimum value of the trend scores, if the first, second, third, and fourth scores have values in descending order.

7. Method according to any one of the preceding claims,
wherein step c) further comprises:
- counting (132b) a number of the trend groups to estimate a number of different tumour sub-clones.

8. Method according to claim 7, further comprising:
- estimating (134b), for each of the sub-clones, a relative fraction of a tumour that it covers, at each point in time.

9. Method according to any of the preceding claims,
wherein the biomarker comprises at least one of circulating tumour DNA, ct-DNA, and genetic analyses of circulating tumour cells, CTCs.

10. Method according to any one of the preceding claims, further comprising:
- outputting at least one of the following into a clinical decision support system:
- the number of different tumour sub-clones;
- for each of the tumour sub-clones, a relative fraction of a tumour that it covers, at each point in time; and/or
- the worst-case trend score.

11. A decision-support apparatus for monitoring a patient's response to a cancer therapy, comprising:
- an input unit configured for receiving data indicative of an occurrence of genetic changes over time in a patient, each of the genetic changes being associated with a common disease or a common disease group, wherein the data is obtained based on an analysis of a biomarker in a patient sample; and
- a processing unit configured for performing a method according to any one of claims 1 to 10.

12. A system (300) for monitoring a patient's response to a cancer therapy, comprising
- a decision-support apparatus according to claim 11; and
- a sample analysing device (310) configured for analysing a patient sample to obtain data indicative of an occurrence of genetic changes over time in the patient to be output to the apparatus.

13. System according to claim 12, further comprising:
- a medical imaging apparatus (320) for monitoring the patient's response to a cancer therapy by imaging.

14. Computer program element for controlling an apparatus according to one of the claims 11 to 13, which, when being executed by a processing unit, is adapted to perform the method steps of any one of claims 1 to 10.

15. Computer readable medium having stored the program element of claim 14.
